# EUROPEAN PATENT SPECIFICATION

(11) **EP 2 662 036 B1**
(45) Date of publication and mention of the grant of the patent: **01.07.2015**
(21) Application number: 12793122.8
(22) Date of filing: 24.05.2012
(51) Int. Cl.: A61B 17/34, A61B 8/12

(54) **PUNCTURE NEEDLE FOR ULTRASOUND**
ULTRASCHALL-PUNKTIONSNADEL
AIGUILLE DE PONCTION POUR ULTRASONS

(30) Priority: 27.05.2011 US 201161490676 P
(43) Date of publication of application: 13.11.2013
(60) Divisional application: 14003230.1
(73) Proprietor: Olympus Medical Systems Corp., Tokyo 151-0072 (JP)
(72) Inventor: SATO Masatoshi, Tokyo 151-0072 (JP)
(74) Representative: Schicker, Silvia
(86) International application number: PCT/JP2012/063382
(87) International publication number: WO 2012/165303

(56) References cited:
- JP-A- 2000 139 926
- JP-A- 2002 306 497
- JP-A- 2002 306 606
- JP-A- 2007 236 684
- US-A1- 2010 099 947

## Description

### Technical Field of the Invention

The present invention relates to a puncture needle for ultrasound biopsy that is inserted into a body cavity and used for delivering medicine and treatment devices into the body.

Priority is claimed on US Provisional Patent Application No. 61/490,676 filed in the US on May 27, 2011.

### Background Art

Hitherto, to inspect and diagnose an affected part in a body cavity, a procedure of sucking and removing body tissue and body fluid has been performed. This procedure is performed by using a puncture needle to pierce the stomach, the duodenal wall, or the like, and puncture a target region of an underlying organ such as the pancreas, the liver, or the kidney while observing the inside of a body cavity through an ultrasonic endoscope. This is termed Endoscopic Ultrasound-guided Fine-Needle Aspiration (EUS-FNA).

Instead of sucking out tissue or body fluid, the EUS-FNA procedure is recently being applied in research into treatment procedures for delivering medicine, a marker, or a substance such as a radioactive source from a puncture needle directly to the region of interest. In such treatment procedures, by delivering the substance accurately to the region of interest, we can expect the effect of the treatment to be enhanced and side-effects to be reduced. It is therefore preferable to perform the procedure while using an ultrasonic endoscope to observe the substance as it is actually being delivered.

US 2010/009947 A1 discloses a fistulectomy method and an ultrasonic endoscope of forming a fistula between a first duct and a second duct. The ultrasonic endoscope comprises an elongated insertion section with a treatment instrument channel into which a puncture needle is introduced. The puncture needle is used to feed a distal end thereof in the second duct through the first duct and to discharge a magnet from the distal end of the puncture needle to the second duct.

### Summary of the Invention

### Problems to be Solved by the Invention

However, the substance to be delivered from a needle tube may not be able to be confirmed on an ultrasonic image in a case where the above treatment in which the puncture needle for EUS-FNA is directly applied is performed. A direction in which the substance is delivered from the needle tube has to be matched to an ultrasonic scan plane in order to confirm the delivery of the substance from the needle tube on the ultrasonic image. For this reason, since an opening part for releasing the substance provided on the needle tube needs to be disposed on the ultrasonic scan plane in a state where the puncture needle is inserted into an insertion channel of the ultrasonic endoscope, it is necessary to regulate an angular position of the needle tube around an axis. However, the ultrasonic endoscope has a long flexible insertion part, and the insertion part is complicatedly curved when the insertion part reaches the target region in the body. Therefore, it is difficult to adjust the angular position of the needle tube, which passes through the insertion part, around the axis by an operation of an operation part at hand. It is also difficult to arrange the opening part for releasing the substance provided on the needle tube on the ultrasonic scan plane at all times. As a result, it may be difficult to accurately deliver the substance to the region of interest since the delivery of the substance cannot be observed with the ultrasonic endoscope.

Because of the above-described problems, a puncture needle for ultrasound biopsy capable of matching an angular position of a needle tube opening part around an axis to an ultrasonic scan plane of an ultrasonic endoscope has been anticipated.

The objective of the present invention is to provide a puncture needle for ultrasound biopsy capable of accurately delivering a substance to a region of interest while the delivery of the substance is favorably being observed with an ultrasonic endoscope.

### Means for Solving the Problem

This object is solved by a puncture needle for ultrasound biopsy used in combination with an ultrasonic endoscope as defined in claim 1. The following means are proposed in the present invention in order to solve the above-described problems.

A puncture needle for ultrasound biopsy includes a sheath, an operation part, a needle tube, and a releasing mechanism, and is used in combination with an ultrasonic endoscope. The sheath is inserted into an insertion channel of the ultrasonic endoscope so as to be capable of advancing and retracting. The operation part may be provided at a proximal-end part of the sheath, and doubles as a gripping part which a user grips. The needle tube is inserted into the sheath, includes an opening formed in a distal-end region thereof, and is configured to puncture a tissue in a body cavity. The releasing mechanism is fitted to a proximal-end side of the needle tube, and releases a substance filled therein from the opening of the needle tube to an outside. Moreover, the needle tube is rotated around a longitudinal axis of the insertion channel by receiving a force from an inner wall of the insertion channel such that an axis line matching a direction of the opening in the needle tube protruding from the insertion channel becomes substantially parallel with an ultrasonic scanning face of the ultrasonic endoscope.

In a natural state, at least a region near a distal end of the needle tube may be curved smoothly in a circular arc, and a direction to visualize a largest area of the opening of the needle tube is substantially parallel with a plane formed by a circular-arc shape of the needle tube.

A center of an outer diameter of the needle tube may be displaced from a center of an inner cavity of the needle tube, and a direction to visualize a largest area of the opening of the needle tube may be substantially parallel with a cross-sectional plane connecting a thinnest part and a thickest part of the needle tube.

An outer-diameter cross-sectional face of the needle tube may have a flat shape, and a direction to visualize a largest area of the opening of the needle tube may be substantially parallel with a short-diameter cross-sectional plane of the flat shape of the needle tube.

In a natural state, at least a region near a distal end of the needle tube may be curved smoothly in a circular arc, and a most proximal-end side of the opening of the needle tube may be in a plane formed by a circular-arc shape of the needle tube.

A center of an outer diameter of the needle tube is displaced from a center of an inner cavity of the needle tube. A most proximal-end side of the opening of the needle tube may be in a cross-sectional plane connecting a thinnest part and a thickest part of the needle tube.

An outer-diameter cross-sectional face of the needle tube may have a flat shape, and a most proximal-end side of the opening of the needle tube may be in a short-diameter cross-sectional plane of the flat shape.

An inner cavity cross-sectional plane of the needle tube may have a flat shape, and a direction to visualize a largest area of the opening may be parallel with a longitudinal direction of the flat shape.

An ultrasonic reflection machining for obtaining a reflected echo may be performed on a distal-end part surface of the needle tube, a pattern of the ultrasonic reflection machining around the opening being different from that in other regions.

Preferably, the distal-end region covers a distal end.

Preferably, the distal-end region covers a region near a distal end.

Preferably, the substance includes gas.

Preferably, the substance includes liquid.

Preferably, the substance includes a solid.

Preferably, the solid includes a slender elastic body.

### Effects of the Invention

According to the puncture needle for ultrasound biopsy, a substance can be accurately delivered to a region of interest while the delivery of the substance is favorably being observed with the ultrasonic endoscope by matching the angular direction position of the needle tube opening part to the ultrasonic scan plane of the ultrasonic endoscope.

### Brief Description of the Drawings

FIG. 1 is an overall view of an ultrasonic endoscope used in combination with a puncture needle for ultrasound biopsy according to a first example.
FIG. 2 is a perspective view of a distal-end part of the ultrasonic endoscope.
FIG. 3 is a front view of the distal-end part of the ultrasonic endoscope.
FIG. 4 is a perspective cross-sectional view of the distal-end part of the ultrasonic endoscope.
FIG. 5 is an overall view of another ultrasonic endoscope used in combination with the puncture needle for ultrasound biopsy according to the first example.
FIG. 6 is a perspective view of a distal-end part of the another ultrasonic endoscope according to the first example.
FIG. 7 is a front view of a distal-end part of the another ultrasonic endoscope.
FIG. 8 is a perspective cross-sectional view of the distal-end part of the another ultrasonic endoscope.
FIG. 9 is an overall exterior view of the puncture needle for ultrasound biopsy according to the first example.
FIG. 10 is an overall cross-sectional view of the puncture needle for ultrasound biopsy according to the first example.
FIG. 11 is an overall cross-sectional view of the puncture needle for ultrasound biopsy according to the first example.
FIG. 12A is a view of a distal-end side of a needle tube of the puncture needle for ultrasound biopsy according to the first example.
FIG. 12B is a view of the distal-end side of the needle tube of the puncture needle for ultrasound biopsy according to the first example.
FIG. 13A is a view of a distal-end side of another example of the needle tube according to the first example.
FIG. 13B is a view of the distal-end side of the another example of the needle tube according to the first example.
FIG. 14A is a view of an implant stored in the needle tube according to the first example and a fifth example.
FIG. 14B is a view of a state where the implant to be stored in the needle tube according to the first example has been stored in the needle tube according to the first example.
FIG. 15 is a view of an operation of the ultrasonic endoscope when the puncture needle for ultrasound biopsy according to the first example is being used.
FIG. 16A is a view of an operation of the puncture needle for ultrasound biopsy according to the first example in a curved ultrasonic endoscope.
FIG. 16B is a view of the operation of the puncture needle for ultrasound biopsy according to the first example in the curved ultrasonic endoscope.
FIG. 16C is a view of the operation of the puncture needle for ultrasound biopsy according to the first example in the curved ultrasonic endoscope.
FIG. 17 is a view of a state where the implant has been delivered from the puncture needle for ultrasound biopsy according to the first example.
FIG. 18 is a view of a state where the puncture needle for ultrasound biopsy according to the first example has been inserted into the another ultrasonic endoscope.
FIG. 19 is an overall schematic view of a puncture needle for ultrasound biopsy according to a second example.
FIG. 20 is an overall cross-sectional view of the puncture needle for ultrasound biopsy according to the second example.
FIG. 21A is a view of a distal-end side of a needle tube of a puncture needle for ultrasound biopsy according to a third example.
FIG. 21B is a view of the distal-end side of the needle tube of the puncture needle for ultrasound biopsy according to the third example.
FIG. 21C is a view of the distal-end side of the needle tube of the puncture needle for ultrasound biopsy according to the third example.
FIG. 22A is an example of an ultrasonic reflection machining performed to the needle tube according to the third example.
FIG. 22B is an example of the ultrasonic reflection machining performed to the needle tube according to the third example.
FIG. 23 is a view of a distal-end side of a needle tube of a puncture needle for ultrasound biopsy according to an embodiment of the present invention.
FIG. 24A is a view of an operation of the puncture needle for ultrasound biopsy according to the embodiment in a curved ultrasonic endoscope.
FIG. 24B is a view of the operation of the puncture needle for ultrasound biopsy according to the embodiment in the curved ultrasonic endoscope.
FIG. 25 is a view of a distal-end side of a needle tube of a puncture needle for ultrasound biopsy according to a fourth example.
FIG. 26A is a view of an operation of the puncture needle for ultrasound biopsy according to the fourth example in a curved ultrasonic endoscope.
FIG. 26B is a view of the operation of the puncture needle for ultrasound biopsy according to the fourth example in the curved ultrasonic endoscope.
FIG. 27 is a view of a distal-end side of a needle tube of a puncture needle for ultrasound biopsy according to the fifth example.
FIG. 28 is a view of a state where an implant has been stored in the needle tube according to the fifth example.

### Embodiment of the Invention

Examples useful to understand the present invention and an embodiment of the present invention are explained with reference to the drawings.

### (First Example)

A puncture needle for ultrasound biopsy according to the present example is used in combination with an ultrasonic endoscope.

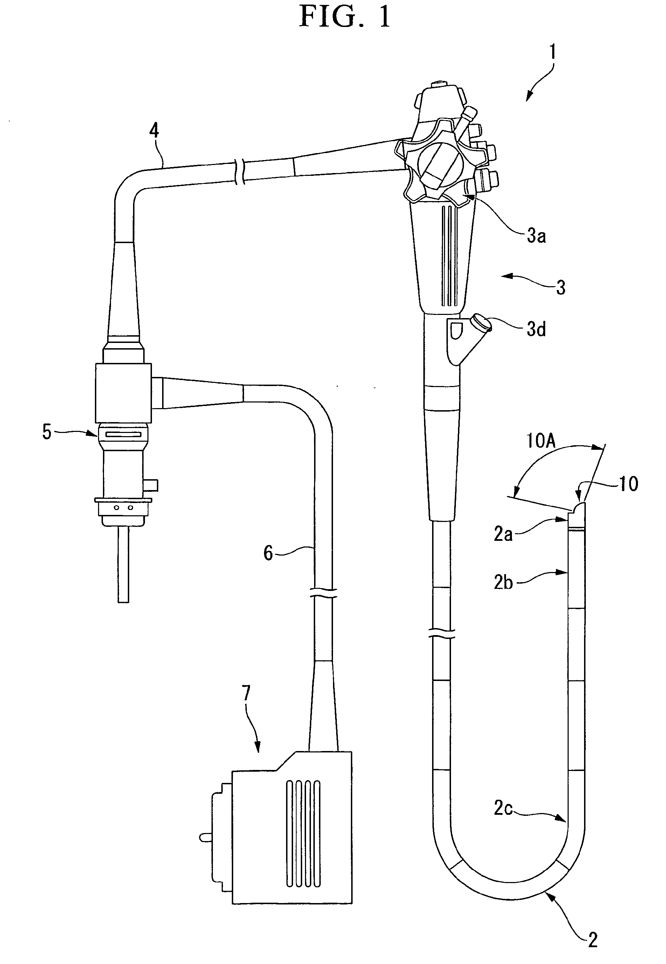

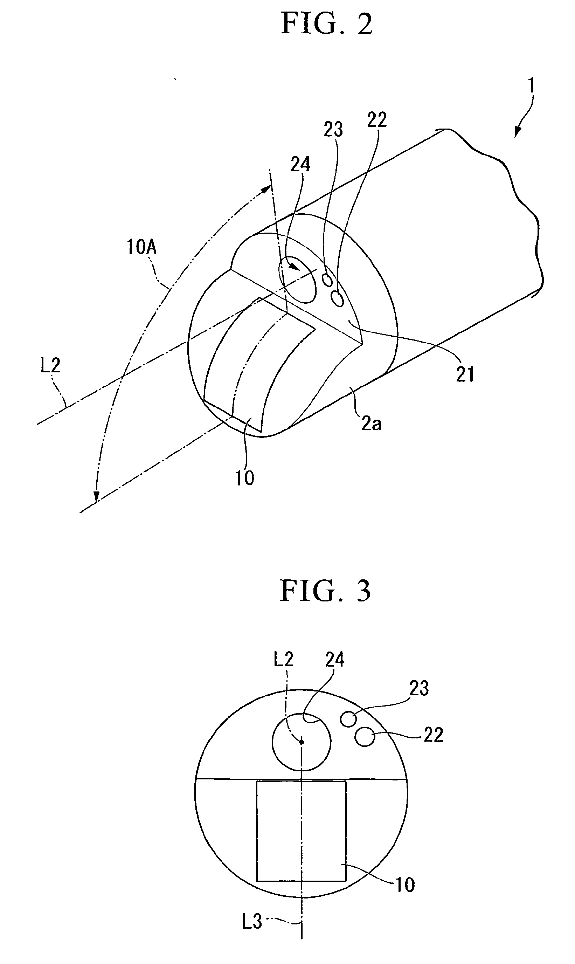

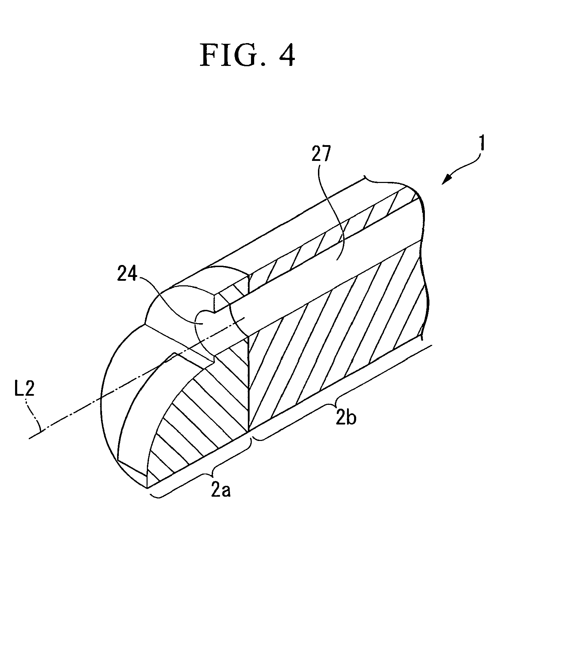

An ultrasonic endoscope according to the present example is explained using FIGS. 1 to 4. FIG. 1 is a view of the configuration of the ultrasonic endoscope. FIG. 2 is a perspective view of a distal-end part of the ultrasonic endoscope. FIG. 3 is a front view of the distal-end part shown in FIG. 2 when seen from the front. FIG. 4 is a perspective cross-sectional view of the distal-end part of the ultrasonic endoscope.

An ultrasonic endoscope 1 includes a slender insertion part 2 for being inserted into a body cavity, an operation part 3 provided at the proximal end of the insertion part 2, and a universal cord 4 that extends from a side part of the operation part 3.

An endoscope connector 5 is provided at a proximal-end part of the universal cord 4. An ultrasonic cable 6 extends from the side part of the endoscope connector 5. An ultrasonic connector 7 is provided at a proximal-end part of the ultrasonic cable 6.

The insertion part 2 includes a distal-end hard part 2a formed from a hard member, a bendable part 2b that can curve, and an elongated flexible tube part 2c that extends from the proximal end of the bendable part 2b to the distal end of the operation part 3, these being connected in that order from the distal-end side.

An ultrasonic transducer part 10 forms an ultrasonic scan plane 10A for scanning in a forward direction with respect to the insertion axis direction. In other words, the ultrasonic transducer part 10 has the ultrasonic scan plane 10A that scans in the forward direction. A signal cable (not shown) is connected to the ultrasonic transducer part 10. This signal cable extends via the insertion part 2, the operation part 3, the universal cord 4, the endoscope connector 5, and the ultrasonic cable 6, to the ultrasonic connector 7.

The ultrasonic connector 7 is connected to an ultrasonic observation device (not shown). The ultrasonic observation device exchanges signals with the ultrasonic transducer via the signal cable, converts a signal received from the ultrasonic transducer to an ultrasonic image, and displays it on a monitor (not shown).

The operation part 3 includes an angle knob 3a for executing a bending operation. The physician manipulates the angle knob 3a as appropriate, whereby a bendable wire (not shown) is pulled or loosened, and the bendable part 2b curves.

As shown in FIG. 2, the ultrasonic transducer part 10 protrudes from a distal-end face 21 of the distal-end hard part 2a of the insertion part 2. Moreover, an observation window 22, an illumination window 23, and an insertion channel exit 24 are provided on the distal-end face 21 of the distal-end hard part 2a. The observation window 22 constitutes the most distal-end side of an observation optical system (not shown). The illumination window 23 constitutes the most distal-end side of an illumination optical system (not shown). The insertion channel exit 24 forms an opening of a treatment tool insertion channel which a treatment tool such as a puncture needle is led through. The insertion channel exit 24 is substantially parallel with the longitudinal axis direction of the distal-end hard part 2a, and is connected to a treatment tool insertion channel (hereinafter abbreviated as 'insertion channel') 27 provided in the insertion part 2 (see

FIG. 4).

The observation optical system and the illumination optical system (both not shown) extend via the insertion part 2, the operation part 3, and the universal cord 4 to the endoscope connector 5. The endoscope connector 5 is connected to an endoscope observation device (not shown). The endoscope observation device transmits illuminating light through the illumination optical system to the illumination window 23, and the illuminating light illuminates the front of the distal-end hard part 2a. The endoscope observation device displays a signal arriving from the observation window 22 via the observation optical system as an observation image on a monitor (not shown). Therefore, the observation image illuminated by the illuminating light is displayed on the monitor.

The proximal-end side of the insertion channel 27 connects to a treatment tool insertion hole 3d provided in the operation part 3. The proximal-end part of the treatment tool insertion hole 3d has a luer-lock shape that allows a syringe to be connected to it. A treatment tool inserted through the treatment tool insertion hole 3d is led out from the insertion channel exit 24.

The center axis L2 of the insertion channel exit 24 is substantially parallel with the longitudinal axis direction of the distal-end hard part 2a. The plane defined by the center axis L2 and the perpendicular direction center line L3 of the ultrasonic transducer part 10 is configured to substantially matches the ultrasonic scan plane 10A. Since the treatment tool led out from the insertion channel exit 24 is led out onto the ultrasonic scan plane 10A, it is visibly displayed on the ultrasonic image.

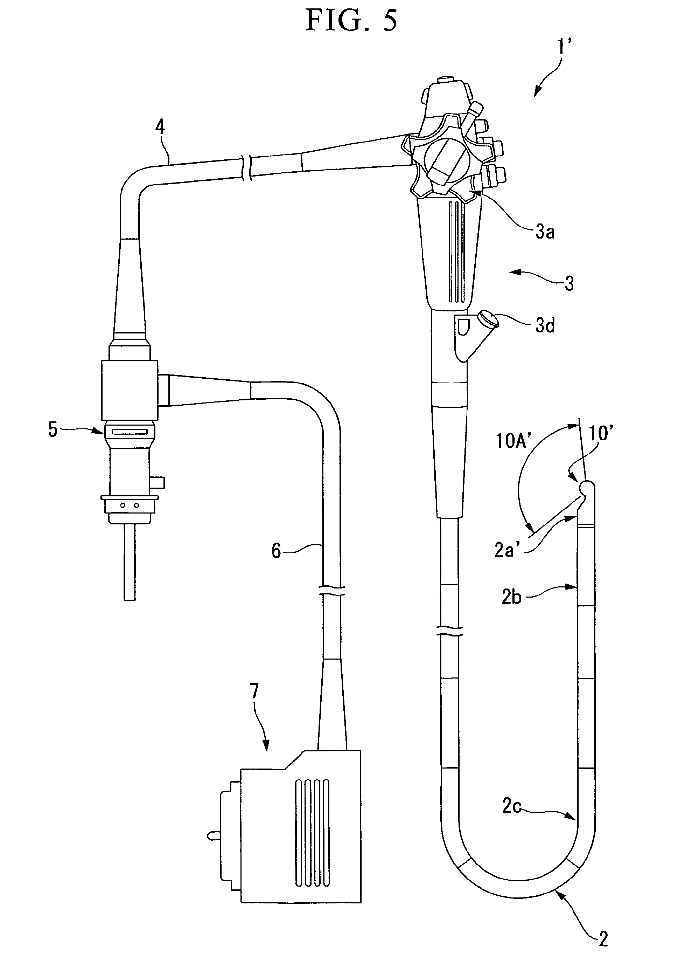

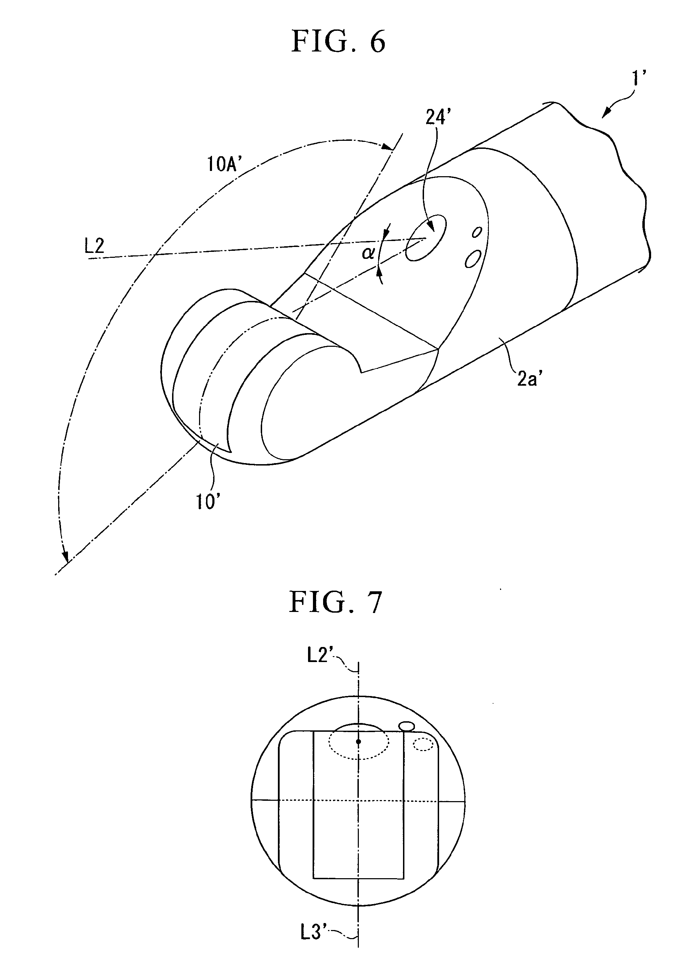

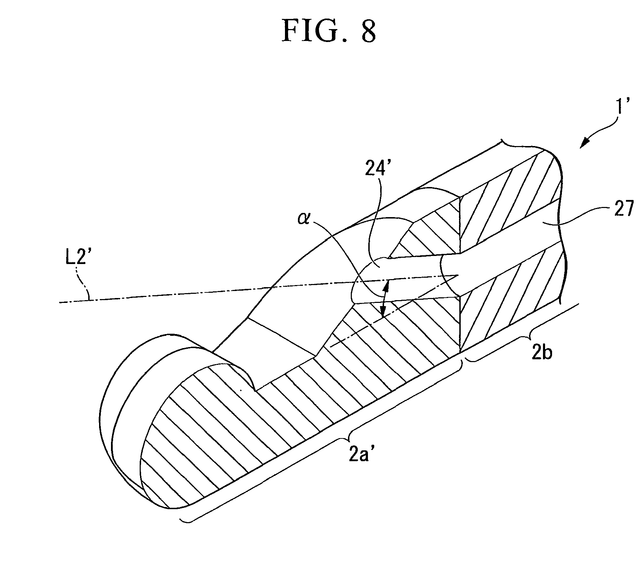

Another ultrasonic endoscope that can be used in the present example is explained using FIGS. 5 to 8. FIG. 5 is a view of the configuration of an ultrasonic endoscope 1'. FIG. 6 is a perspective view of a distal-end part of the ultrasonic endoscope 1'. FIG. 7 is a front view of the distal-end part shown in FIG. 5 when seen from the front face. FIG. 8 is a perspective cross-sectional view of the distal-end part of the ultrasonic endoscope 1'.

In the ultrasonic endoscope 1', sections having a configuration similar to that of the ultrasonic endoscope 1 described above are designated with the same reference numerals in FIGS. 5 to 8. A point of difference with the ultrasonic endoscope 1 is that the ultrasonic transducer part 10' at the distal end is larger. As a result, the ultrasonic scan plane 10A' that scans in a forward direction with respect to the insertion axis direction has a wider field of view.

Moreover, as shown in FIG. 6, the insertion channel exit 24' of the ultrasonic endoscope 1' is tilted at an angle α with respect to the longitudinal axis direction of the distal-end hard part 2a', such that the treatment tool led out from the insertion channel exit does not make contact with the large ultrasonic transducer part 10'.

However, the plane defined by the longitudinal direction center axis L2' of the insertion channel exit 24' and the perpendicular direction center line L3' of the ultrasonic transducer 10' is configured to be substantially matched to the ultrasonic scan plane 10A'. This configuration is the same as the ultrasonic endoscope 1. The treatment tool led out from the insertion channel exit 24' is therefore led out onto the ultrasonic scan plane 10A' and visibly displayed on the ultrasonic image.

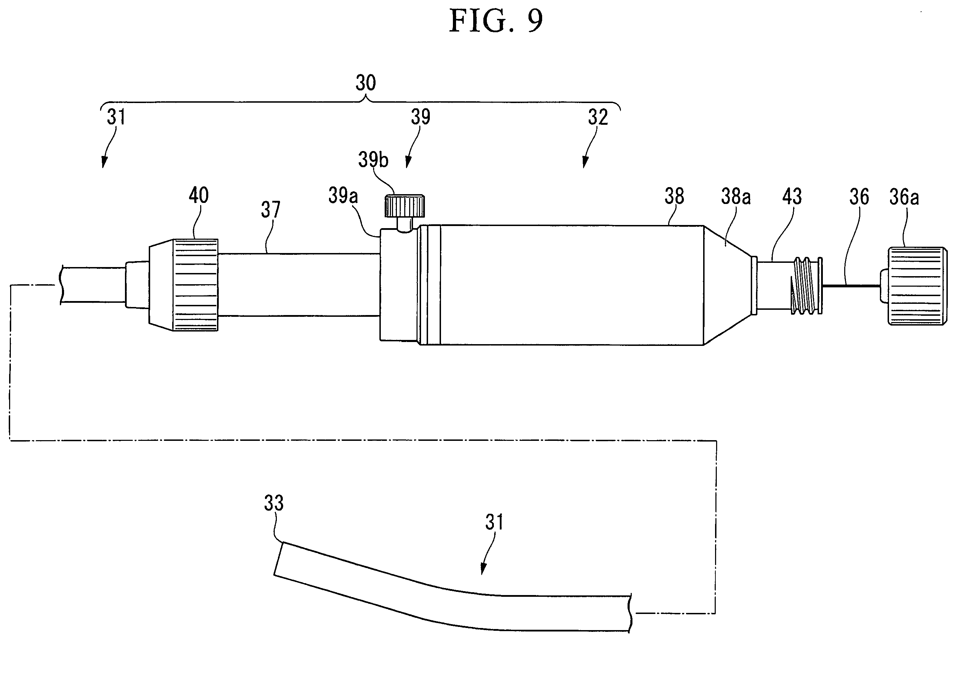

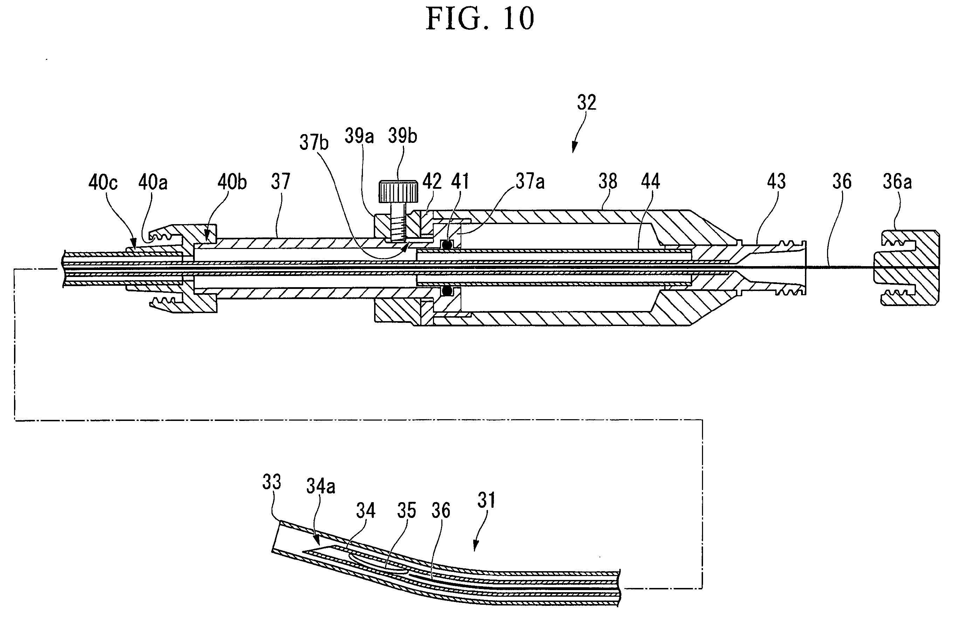

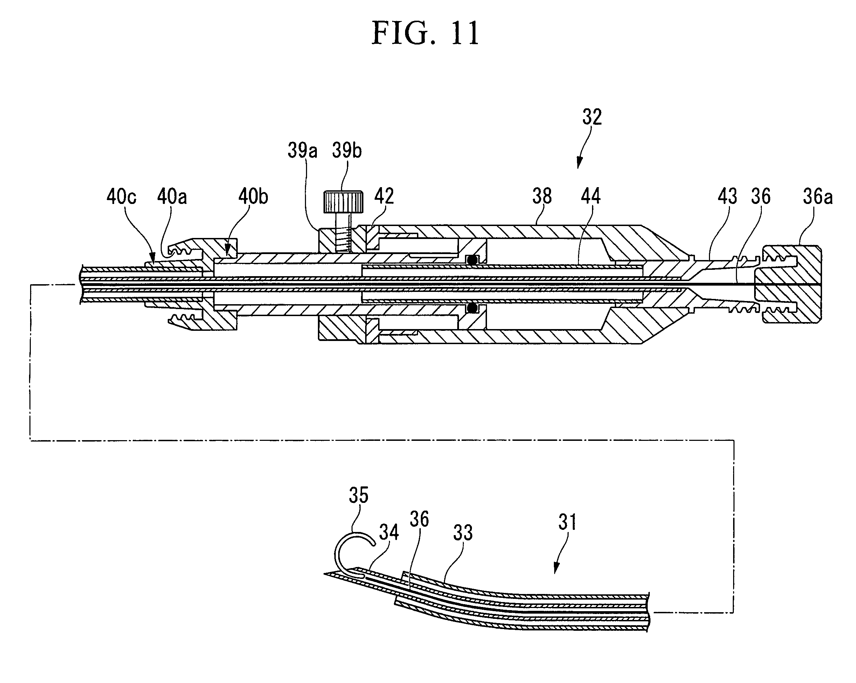

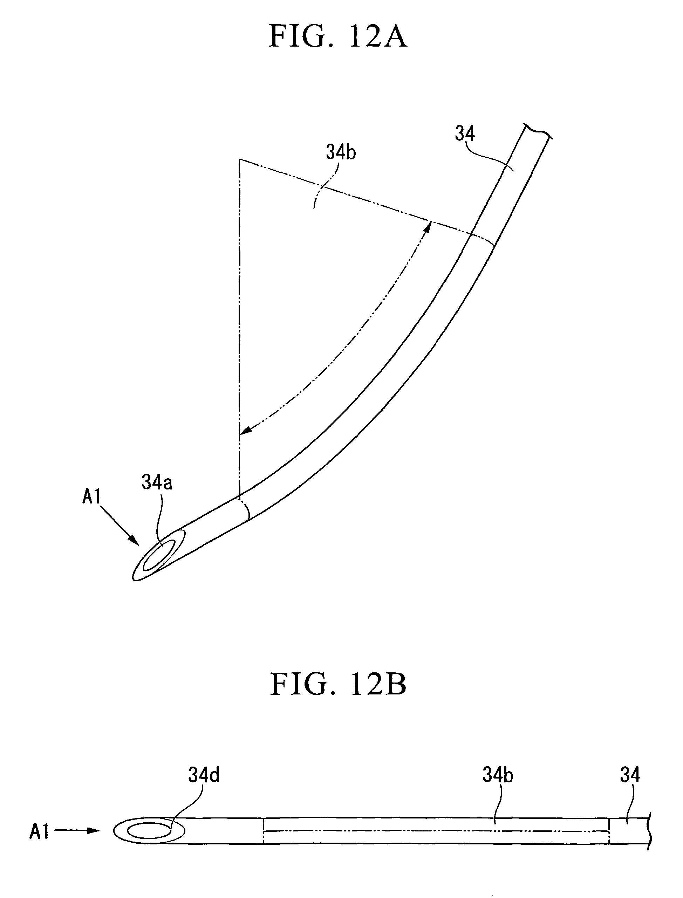

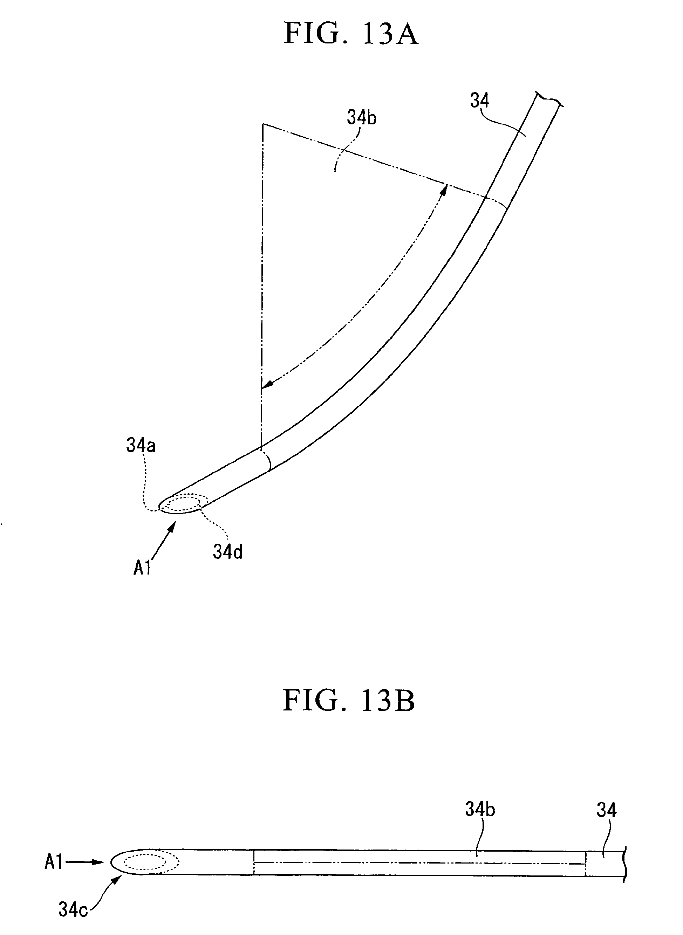

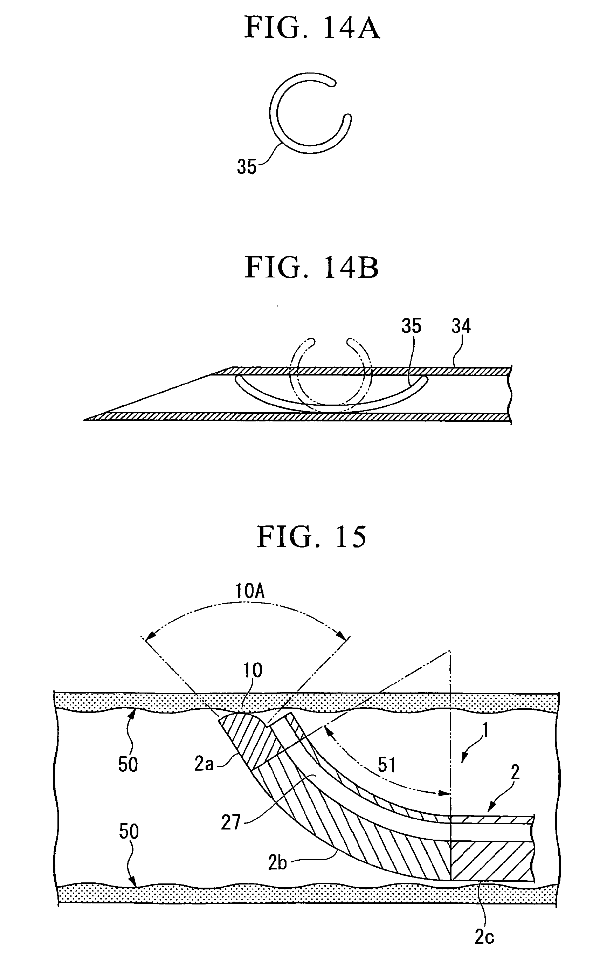

Next, a puncture needle for ultrasound biopsy according to the present example is explained using FIGS. 9 to 14B. FIG. 9 is an overall exterior view of the puncture needle for ultrasound biopsy. FIGS. 10 and 11 are overall cross-sectional views. FIGS. 12A, 12B, 13A and 13B are views for explaining the shape of a needle tube. FIGS. 14A and 14B are explanatory views of an implant.

A puncture needle for ultrasound biopsy 30 according to the present example includes an insertion part 31 and an operation part 32. The insertion part 31 is the section that is inserted into the insertion channel 27 of the ultrasonic endoscope 1. The operation part 32 is provided at the proximal-end part of the insertion part 31, and is secured to the treatment tool insertion hole 3d of the ultrasonic endoscope 1.

Each part of the insertion part 31 is explained.

A sheath 33 is a flexible tube, and is disposed on the outermost side of the insertion part 31. A resin such as, for example, polyetheretherketone, polyethersulfone, or Teflon (Registered Trademark) is suitable as the material for the sheath 33. Alternatively, a metal wire conventionally known as a flexible shaft, particularly a wound metal formed by winding a stainless steel wire in the shape of a coil spring, is suitable as the material for the sheath 33. A needle tube 34 is inserted into an inner cavity of the sheath 33. The needle tube 34 and the insertion channel 27 are prevented from being damaged by direct contact between the needle tube 34 and the inner face of the insertion channel 27.

The needle tube 34 is formed from, for example, a slender and thin stainless steel pipe or such like, and has a sharp distal end. The needle tube 34 is inserted into the sheath 33 such that it can advance/retract.

FIGS. 12A to 13B show the region near the distal end of the needle tube 34 in greater detail. When the needle tube 34 is in a natural state, at least the region near the distal end is curved smoothly in a circular arc. While the most distal end of the needle tube 34 is not curved in a circular arc in FIGS. 12A and 12B, it can be included within the section that is curved in a circular arc.

The distal end of the needle tube 34 is cut into a diagonal shape like a conventional syringe, and an inner cavity is open in the diagonally cut face. The direction perpendicular to the distal-end face, i.e. the direction to visualize the largest area of the opening 34a (indicated by arrow A1 in FIG. 12A), is substantially parallel with the plane 34b that contains the longitudinal center axis of the needle tube 34. A point 34d at the most proximal-end side of the opening 34a is coplanar with the plane 34b.

In the example shown in FIGS. 13A and 13B, the distal end of the needle tube 34 is cut in the reverse direction to the example shown in FIGS. 12A and 12B; either can be selected depending on the objective.

An implant 35 is a metal piece containing a substance that generates very weak radiation for treatment purposes. FIGS. 14A and 14B show the implant 35 in greater detail. Its shape is formed by bending a rod-like material that is more slender than the inner cavity of the needle tube 34, obtaining a rod spring with elasticity. The implant 35 filled into the distal end of the inner cavity of the needle tube 34 has elasticity, and thus pushes the inner wall of the needle tube 34 with a force of attempting to return to its former shape. Therefore, it is not easily dislodged from the needle tube 34.

A stylet 36 is a slender wire. The stylet 36 is made from, for example, stainless steel or nickel titanium. The stylet 36 is disposed on the proximal-end side of the inner cavity of the needle tube 34 so that it can be inserted and removed. By an operation described below, the stylet 36 pushes the implant 35 out from the needle tube 34.

Constitutive elements of the operation part 32 are explained.

An operation-part main unit 37 is formed from a resin member.

A slider 38 is provided so as to slide with respect to the operation-part main unit 37. The slider 38 is formed from a resin member.

A stopper 39 is a member configured such that a sliding distance of the slider 38 with respect to the operation-part main unit 37 can be set at a desired value in accordance with a result of measuring the sliding distance, and is configured as follows. A stopper member 39a is disposed so as to slide with respect to the operation-part main unit 37. The stopper member 39a is made from, for example, a resin. A fixing screw (a stopper screw) 39b is screwed into the stopper member 39a, and fix the stopper member 39a in a desired position. The fixing screw 39b is made from metal or a hard resin.

The operation-part main unit 37 is shaped like a slender pipe, with a flange part 37a at its proximal-end part. A resin connection part 40 fixed to the proximal-end part of the sheath 33 is adhesively fixed to a distal-end part of the operation-part main unit 37. On the distal-end side of the connection part 40, a screw 40a, which is fixed and connected to the treatment tool insertion hole 3d of the ultrasonic endoscope 1, is formed. The proximal-end part of the connection part 40 includes a recess 40b in which the distal-end part of the operation-part main unit 37 is disposed. The sheath 33 is fixed to a distal-end connection part 40c formed in the connection part 40.

A recess is formed in the inner peripheral face of the flange part 37a. An O-ring 41 which holds a guide pipe described below is disposed in the recess in the flange part 37a. A notched step 37b which has a flat-faced part that the fixing screw 39b contacts is formed at a predetermined position on the outer peripheral face further to the distal-end side than the flange part 37a.

At the time of manufacture and shipment of the puncture needle for ultrasound biopsy 30, the distal-end face of the fixing screw 39b is in contact with the flat-faced part of the notched step 37b at a predetermined torque, whereby the slider 38 is arranged on the proximal-end side of the operation-part main unit 37.

In this arrangement state, the distal-end parts of the needle tube 34 and the stylet 36 are arranged inside the sheath 33. Even if the slider 38 is moved to the distal-end side due to some kind of external force, the side part of the fixing screw 39b contacts the rising part of the notched step 37b and stops the slider 38 from moving toward the distal-end side. In this contacting state, the distal-end parts of the needle tube 34 and the stylet 36 do not, of course, protrude from the distal end of the sheath 33.

When the fixing screw 39b is loosened, the stopper member 39a can slide on the operation-part main unit 37 in the longitudinal direction. The maximum movable distance of the slider can be set by sliding the stopper member 39a to a given position, and fixing it by screwing in the fixing screw 39b.

The slider 38 is shaped like a pipe with a small-diameter part 38a at its proximal-end part. A sliding arrangement member 42 to arrange the slider 38 so as to slide with respect to the operation-part main unit 37 is adhesively fixed to the distal-end part of the slider 38.

On the other hand, a resin ferrule member 43 is disposed in the proximal-end opening part of the slider 38. The proximal-end part of the needle tube 34, and the proximal-end part of a guide pipe 44 having a distal-end part held by the O-ring 41, are fixed to the distal-end part of the ferrule member 43. The proximal-end part of the ferrule member 43 has a luer-lock shape that allows a syringe or the like to be connected to it.

The stylet 36 is inserted from the ferrule member of the slider 38. A resin knob 36a is provided in a single piece at the proximal-end part of the stylet 36.

The puncture needle for ultrasound biopsy 30 having the above configuration is stored in a sterilization bag (not shown) and disinfected after the constitutive members are assembled.

An operation of the disposable type of the puncture needle for ultrasound biopsy 30 having the configuration described above is explained. First, a case where the ultrasonic endoscope 1 shown in FIGS. 1 to 4 is combined with the needle tube 34 shown in FIGS. 12A and 12B is explained. Next, a case that functions in exactly the same way by using the ultrasonic endoscope 1' shown in FIGS. 5 to 8 and the needle tube 34 shown in FIGS. 13A and 13B is explained.

Firstly, the physician takes the puncture needle for ultrasound biopsy 30 stored in a sterilization bag (not shown) out of the sterilization bag. The physician then inserts the sheath 33 from the treatment tool insertion hole 3d of the ultrasonic endoscope 1 into the insertion channel 27, screws the screw 40a provided at the connection part 40 of the operation part 32 into the treatment tool insertion hole 3d, and thus fixes the puncture needle for ultrasound biopsy 30 to the ultrasonic endoscope 1.

As a result, an ultrasonic image of the distal-end part of the sheath 33 is clearly depicted on an ultrasonic observation image showing the target region. Here, the physician sets the positional relationship between the distal end of the sheath 33 and the target region. Then, the physician measures the distance between them.

Next, the physician loosens the fixing screw 39b, and slides the stopper member 39a on the operation-part main unit 37 in correspondence with the above-mentioned distance. When the physician has moved the stopper member 39a to a predetermined position, the physician tightens the fixing screw 39b.

Thereafter, the physician grips the slider 38, and moves it rapidly toward the stopper 39. The tip of the needle tube 34 thus reliably pierces the target region.

After checking that the needle tube 34 has reached the target region, the physician pushes the knob 36a of the stylet 36 to the distal-end side. The stylet 36 is moved to the distal-end side, and the implant 35 is delivered from the opening 34a at the distal end of the needle tube 34, and is deposited inside the body.

To deposit the implant 35 accurately in the target region, the physician must check the implant 35 on the ultrasonic observation image while the physician delivers it. Accordingly, in the example, the angular position of the needle tube 34 around the axis is controlled so as to match the direction of delivering the implant 35 with the ultrasonic scan plane. A method of matching the direction of delivering the implant 35 with the ultrasonic scan plane is explained below.

Since ultrasonic waves attenuate severely in air, during ultrasonic observation, the ultrasonic transducer part 10 disposed at the distal end of the ultrasonic endoscope 1 must be brought firmly into contact with the body tissue. In FIG. 15, since the ultrasonic endoscope 1 is inserted into the body-cavity tissue 50, to bring the ultrasonic transducer 10 firmly into contact with the tissue, the bendable part 2b of the insertion part 2 must be curved in the direction normally termed 'up' and directed toward the body-cavity tissue 50. When it has been curved, the bendable part 2b is shaped substantially like a circular arc, and, naturally, so is the insertion channel 27 disposed inside it. The plane 51 containing the longitudinal center axis of the insertion channel 27 is now substantially coplanar with the ultrasonic scan plane 10A.

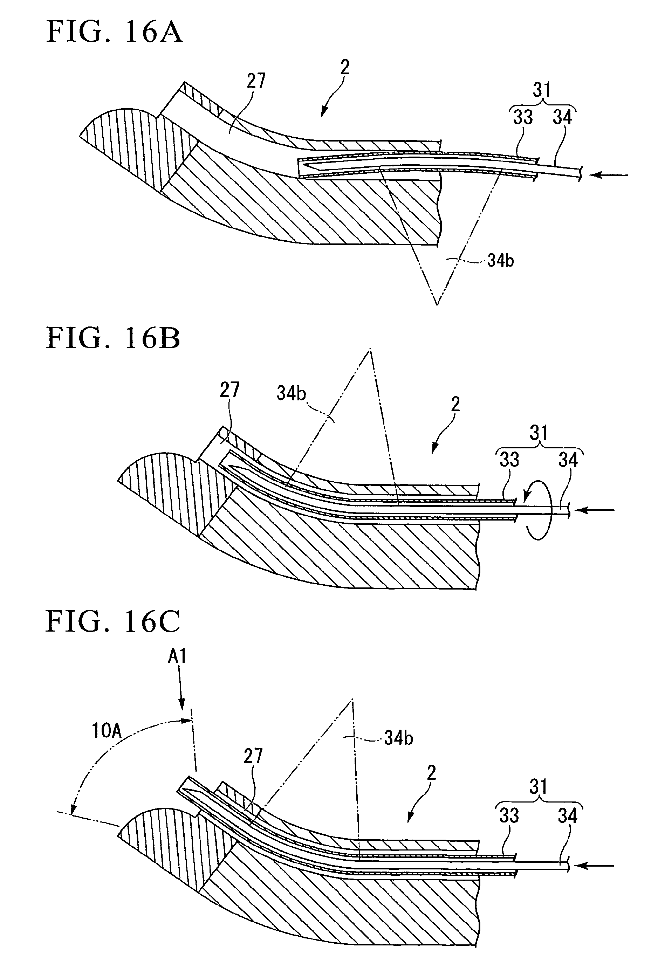

FIGS. 16A to 16C illustrate the state of the insertion part 31 of the puncture needle for ultrasound biopsy 30, including the needle tube 34 with the section near its distal end curved smoothly in a circular-arc shape, being passed through the curved insertion channel 27 in chronological sequence of the operation. In FIG. 16A, the distal end of the insertion part 31 has been pushed to just before the curved part of the insertion channel 27 of the ultrasonic endoscope 1. When the insertion part 31 is pushed further, as shown in FIG. 16B, the circular-arc shape of the needle tube 34 reaches the curved shape of the insertion part 2. As it is inserted, the needle tube 34 receives a force from the inner wall of the insertion channel 27, and the needle tube 34 is consequently made to rotate around the longitudinal axis, such that the circular-arc shape of the insertion channel 27 and the circular-arc shape of the needle tube 34 are coplanar (including substantially coplanar). As a result, the plane 34b containing the longitudinal center axis of the needle tube 34 (i.e. the plane containing an axis that matches the direction of the opening in the needle tube 34) becomes the same as the plane 51 containing the long center axis of the insertion channel 27. Therefore, the plane 34b becomes substantially coplanar with the ultrasonic scan plane 10A.

FIG. 16C illustrates the state when the insertion part 31 has reached the predetermined position. While the angular position of the needle tube 34 around the axis is unchanged from that in FIG. 16B, the length of the overlap between the circular-arc shape of the needle tube 34 and the curved shape of the insertion part 2 increases, and the angular position of the needle tube 34 around the axis is therefore more stable. Since the direction A1 for visualizing the largest area of the opening 34a is substantially parallel with the plane 34b, the direction A1 becomes substantially parallel with the ultrasonic scan plane 10A. In other words, the axis matching the direction of the opening in the needle tube 34 becomes substantially parallel with the ultrasonic scan plane 10A.

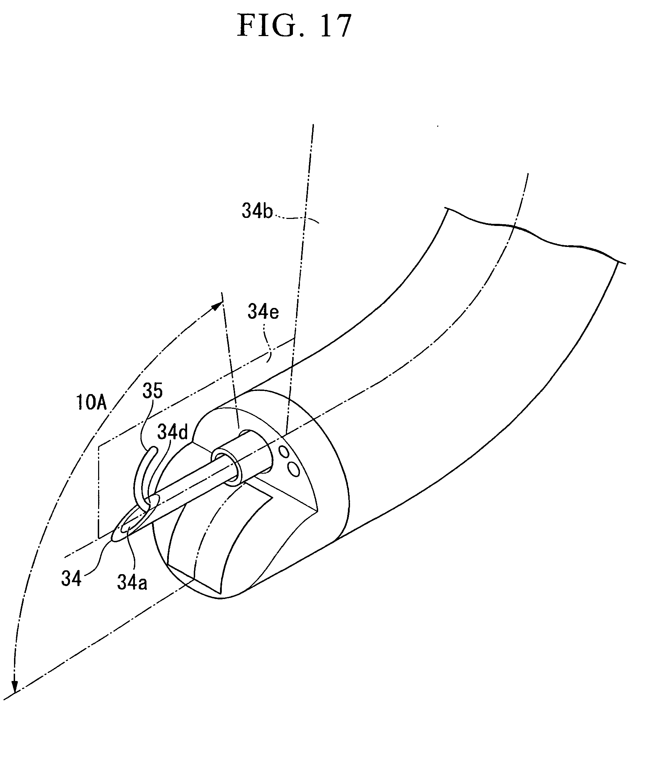

As mentioned earlier, the implant 35 has elasticity, and is an extended state while being stored inside the needle tube 34. As shown in FIG. 17, when the implant 35 is delivered from the opening 34a of the needle tube 34, it attempts to return from the extended state to its original shape. The implant 35 consequently rubs against the point 34d at the most proximal-end side of the opening 34a as it is delivered. The implant 35 is now delivered within a plane 34e. The plane 34e is substantially the same as the plane 34b and contains the proximal-end point 34d.

Since the plane 34b is substantially the same as the ultrasonic scan plane 10A, the implant 35 can be favorably confirmed on the ultrasonic image.

Next, a case using the ultrasonic endoscope 1' shown in FIGS. 5 to 8 and the needle tube 34 shown in FIGS. 13A and 13B is explained.

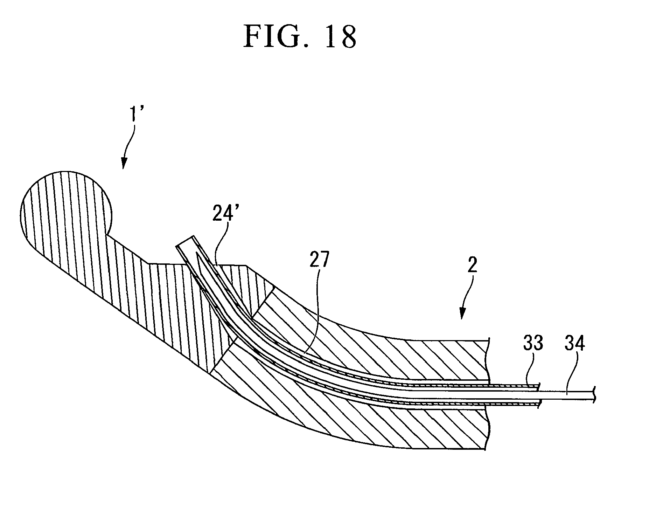

FIG. 18 is a state where the insertion part 31 of the puncture needle for ultrasound biopsy 30, including the needle tube 34 with a part near the distal-end that is curved in a circular-arc shape in a natural state, has been passed along the curved insertion channel 27, and has reached a predetermined position.

As shown in FIG. 6, the insertion channel exit 24' tilts at an angle α with respect to the longitudinal axis direction of the distal-end hard part 2a', such that the treatment tool led out from the insertion channel exit 24' does not make contact with the large ultrasonic transducer part 10'. As shown in FIG. 15, during the actual procedure, the bendable part 2b of the endoscope insertion part 2 is curved in the direction normally termed 'up'. It is clear that, in this state, the needle tube 34 curved in a circular-arc shape can be smoothly passed through the cavity formed by the insertion channel exit 24' and the insertion channel 27. Therefore, when the implant 35 is delivered from the opening 34a of the needle tube 34, it can be favorably confirmed on the ultrasonic image.

Of course, when the ultrasonic endoscope 1 shown in FIGS. 1 to 4 is used in combination with the needle tube shown in FIGS. 13A and 13B, and when the ultrasonic endoscope 1' shown in FIGS. 5 to 8 is used in combination with the needle tube shown in FIGS. 12A and 12B, similar functions are achieved. After the implant 35 has been ejected into the body, the puncture needle for ultrasound biopsy 30 is detached from the ultrasonic endoscope and discarded, and this series of procedures ends.

### (Second Example)

In the first example, the case where a solid implant is deposited inside a body is described. An alternative case where a gaseous or liquid medicine is injected into the body is explained as a second example. In the following description, sections similar to those in the configuration in the first example are not repetitiously explained, and only points of difference are explained.

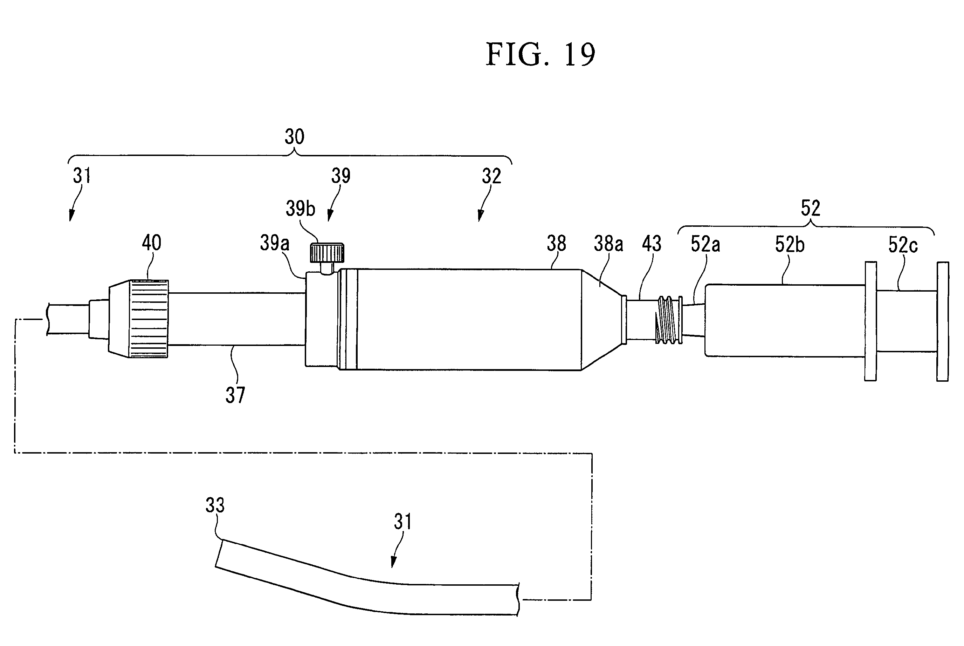

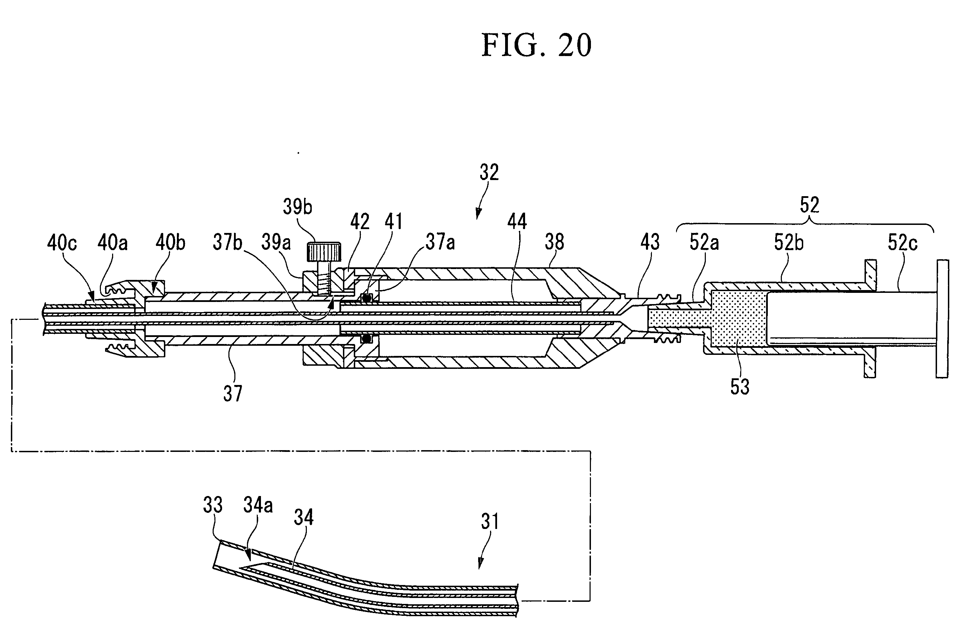

A puncture needle for ultrasound biopsy used in the present example is explained using FIGS. 19 and 20. FIG. 19 is an overall schematic view of the puncture needle for ultrasound biopsy. FIG. 20 is an overall cross-sectional view of the puncture needle for ultrasound biopsy.

An injection syringe 52 is attached to a resin ferrule member 43. The injection syringe 52 includes a cylinder ferrule 52a, a cylinder 52b, and a piston 52c, and the cylinder 52b is filled with a gaseous or liquid medicine 53.

The mechanism for controlling the angular position of the needle tube 34 around the axis is similar to that of the first example, and is not repetitiously explained.

When the piston 52c of the injection syringe 52 is pressed, the medicine 53 passes along the inner cavity of the needle tube 34 and is released into the body from the opening 34a, whereby the medicine 53 is released onto the ultrasonic scan plane 10A. Therefore, it is possible to favorably confirm on the ultrasonic image that the medicine 53 is being delivered.

### (Third Example)

A configuration in which the distal-end shape of the needle tube differs from that in the first and the second examples is explained as a third example. In the present example, the substance being delivered into the body can be a solid such as the implant 35, or it can be a gas or liquid such as the medicine 53. Other than the distal-end shape of the needle tube, the puncture needle for ultrasound biopsy according to the present example has the same configuration as the first example when delivering a solid. In addition, other than the distal-end shape of the needle tube, the puncture needle for ultrasound biopsy according to the present example has the same configuration as the second example when delivering a gas or liquid such as the medicine 53. Therefore, only points of difference in the configuration from the first and the second examples are described.

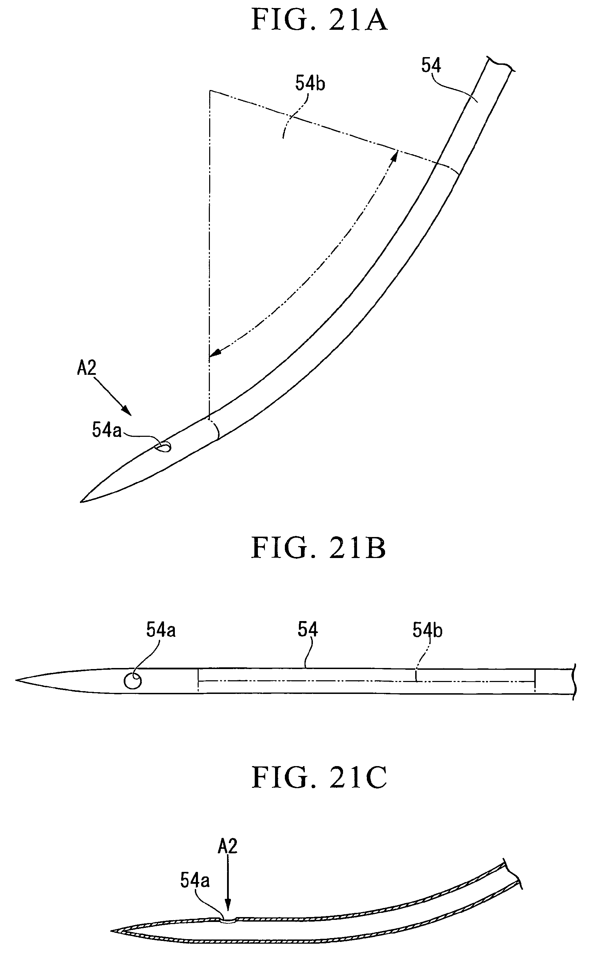

As shown in FIGS. 21A to 21C, the distal end of the needle tube 54 is sharp, and an opening 54a is provided in its side face. The direction to visualize this opening 54a from the front, i.e. the direction to visualize the largest area (indicated by arrow A2 in FIGS. 21A and 21C) is substantially parallel with the plane 54b that contains the longitudinal center axis of the needle tube 54. While the opening 54a is one in this example, openings can be appropriately provided in accordance with the desired dispersal range of the medicine. For example, another opening can be provided at a position 180-degree opposite to the opening 54a. In addition, a plurality of openings can be provided along the longitudinal direction of the needle tube 54.

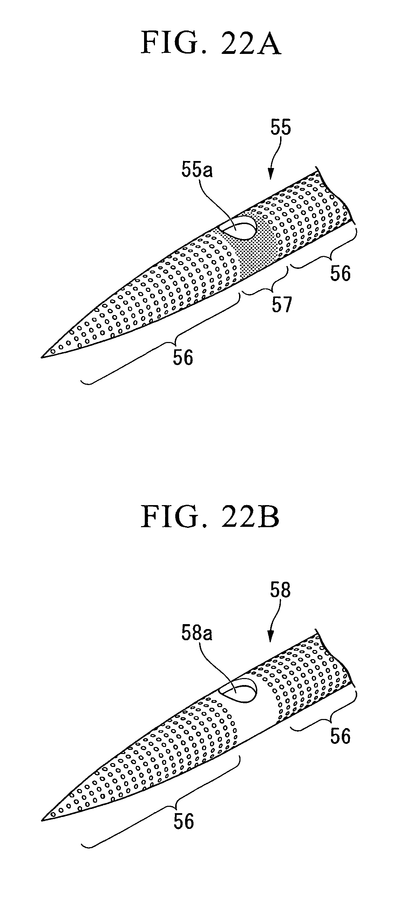

With a conventional puncture needle for ultrasound biopsy, ultrasonic reflection machining is sometimes used to enhance the visibility of the needle tube on the ultrasonic image. In ultrasonic reflection machining, a great many minute recesses such as dimples are formed on the surface of the needle tube, or the surface is made rougher. With regard to this ultrasonic reflection machining, the needle tube 54 is configured such that a pattern of ultrasonic reflected from near the opening 54a is different from that of ultrasonic reflected from the distal-end side and proximal-end side of the opening 54a. Specific examples of such ultrasonic reflection are shown in FIGS. 22A and 22B.

In FIG. 22A, ultrasonic reflection machining 57 is performed around an opening 55a in a needle tube 55. Ultrasonic reflection machining 56 with a different ultrasonic reflectivity to the ultrasonic reflection machining 57 is performed at the front and rear of the opening 55a in the longitudinal direction. In FIG. 22B, machining is not performed to the surface around an opening 58a in a needle tube 58, and ultrasonic reflection machining 56 is performed at the front and rear of the opening 58a in the longitudinal direction, so that the ultrasonic reflection pattern is different.

By changing the reflectivity of ultrasonic near the opening and on the distal-end side and proximal-end side of the opening in this way, the brightness of the region near the opening on the ultrasonic image is different from the brightness of the distal-end side and proximal-end side, enabling the position of the opening to be confirmed clearly. Therefore, a substance can be supplied more accurately. The ultrasonic reflection machining formed at the front and rear of the opening in the longitudinal direction need only have a different ultrasonic reflectivity to the region around the opening, and is not limited to the pattern indicated by the ultrasonic reflection machining 56.

In the present example, the mechanism for controlling the angular position of the needle tube around the axis is similar to that of the first example, and is not repetitiously explained.

### (Embodiment of the present invention)

An example where the configuration of the needle tube is different from that in the first to the third examples is explained as an embodiment of the present invention. In the present embodiment, the substance being delivered into the body can be a solid such as the implant 35, or it can be a gas or liquid such as the medicine 53. Other than the configuration of the needle tube, the puncture needle for ultrasound biopsy according to the present embodiment has the same configuration as the first example when delivering a solid. In addition, other than the configuration of the needle tube, the puncture needle for ultrasound biopsy according to the present embodiment has the same configuration as the second example when delivering a gas or liquid such as the medicine 53. Therefore, only points of difference in the configuration from the first to the third examples are described.

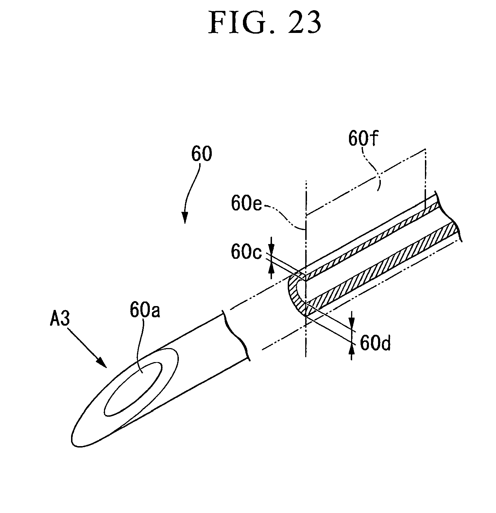

In a needle tube 60 shown in FIG. 23, the center of the outer diameter thereof is displaced from the center of the inner cavity thereof, and an opening 60a is formed at the distal end thereof. The opening 60a is provided such that the direction to visualize the largest area of the opening 60a (indicated by arrow A3 in FIG. 23) is substantially parallel with a longitudinal cross-sectional plane 60f that contains a line 60e connecting the thinnest part 60c and the thickest part 60d of the needle tube. In a natural state where no external force is acting on it, the needle tube 60 maintains a linear shape (including a substantially linear shape).

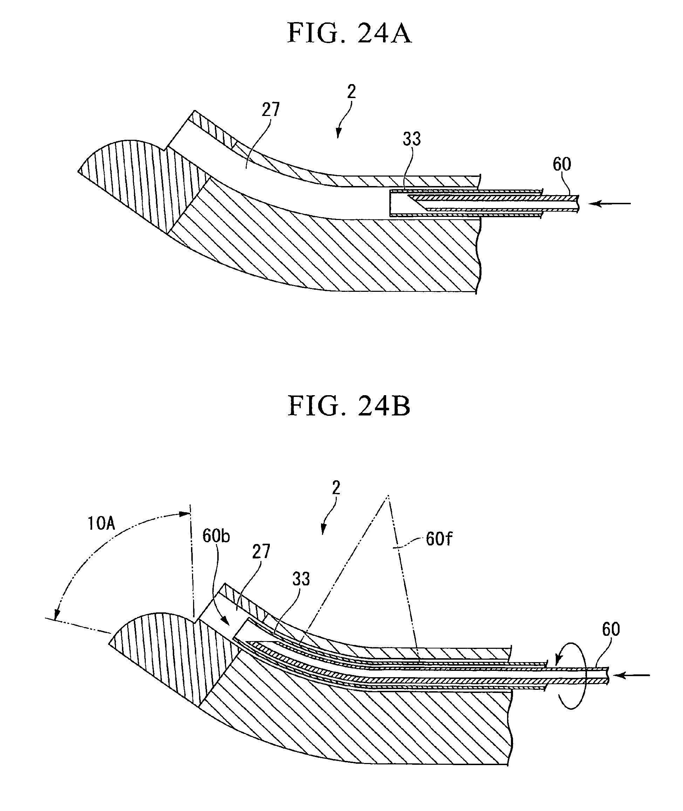

As shown in FIGS. 24A and 24B, when the insertion part of the puncture needle for ultrasound biopsy, containing the needle tube 60 with the outer diameter eccentric to the inner cavity, is passed into the insertion channel 27 of the curved ultrasonic endoscope 1, the needle tube 60 is made to rotate around the longitudinal axis such that the thinnest part of the needle tube 60 is disposed on the inner side of the curve of the ultrasonic endoscope 1. As a result, the longitudinal cross-sectional plane 60f that contains the line 60e connecting the thinnest and thickest parts of the needle tube 60 becomes substantially the same as the ultrasonic scan plane 10A. Therefore, the direction A3 to visualize the largest area of the opening 60a becomes substantially parallel with the ultrasonic scan plane 10A. The substance that has been delivered can thus be favorably confirmed on the ultrasonic image.

### (Fourth Example)

An example where the configuration of the needle tube differs from that in the first to the third examples and from the embodiment of the present invention is explained as a fourth example. In the present example, the substance being delivered into the body can be a solid such as the implant 35, or it can be a gas or liquid such as the medicine 53. Other than the configuration of the distal end of the needle tube, the puncture needle for ultrasound biopsy according to the present example has the same configuration as the first example when delivering a solid. In addition, other than the configuration of the distal end of the needle tube, the puncture needle for ultrasound biopsy according to the present example has the same configuration as the second example when delivering a gas or liquid such as the medicine 53. Therefore, only points of difference in the configuration from the first to third examples and from the embodiment of the present invention are described.

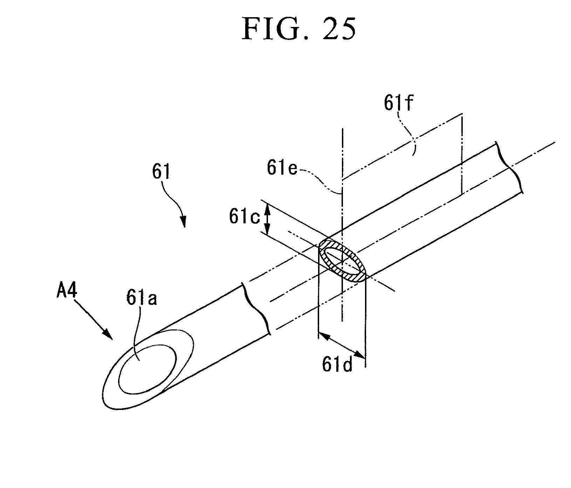

A needle tube 61 shown in FIG. 25 has a flat cross-sectional shape, such as an ellipse, an oval, or a rectangle, and the direction to visualize the largest area of an opening 61a in the needle tube 61 (indicated by arrow A4 in FIG. 25) is substantially parallel with a longitudinal cross-sectional plane 61f that contains the short-diameter center axis 61e of the flat shape of the needle tube.

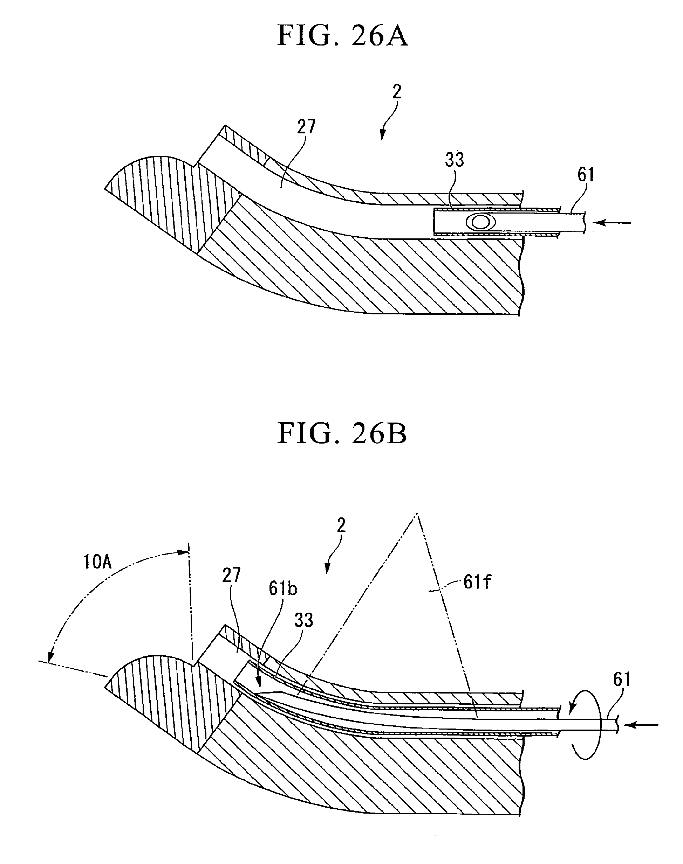

As shown in FIGS. 26A and 26B, when the insertion part of the puncture needle for ultrasound biopsy, containing the needle tube 61 with the flat cross-sectional shape, is passed into the insertion channel 27 of the curved ultrasonic endoscope 1, the needle tube 61 is made to rotate around the longitudinal axis in a direction such that the short-diameter side faces the center of the curve. As a result, the longitudinal cross-sectional plane 61f that contains the short-diameter center axis 61e of the needle tube becomes substantially parallel with the ultrasonic scan plane 10A. Therefore, the direction A4 to visualize the largest area of the opening 61a becomes substantially parallel with the ultrasonic scan plane 10A. Thus, the substance that has been delivered can be favorably confirmed on the ultrasonic image.

### (Fifth Example)

An example where the configuration of the needle tube differs from that in the first example is explained as a fifth example. In the present example, the substance being delivered into the body is a solid such as the implant 35. Accordingly, other than the configuration of the distal end of the needle tube, the present example has the same configuration as the first example, and therefore only points of difference are described.

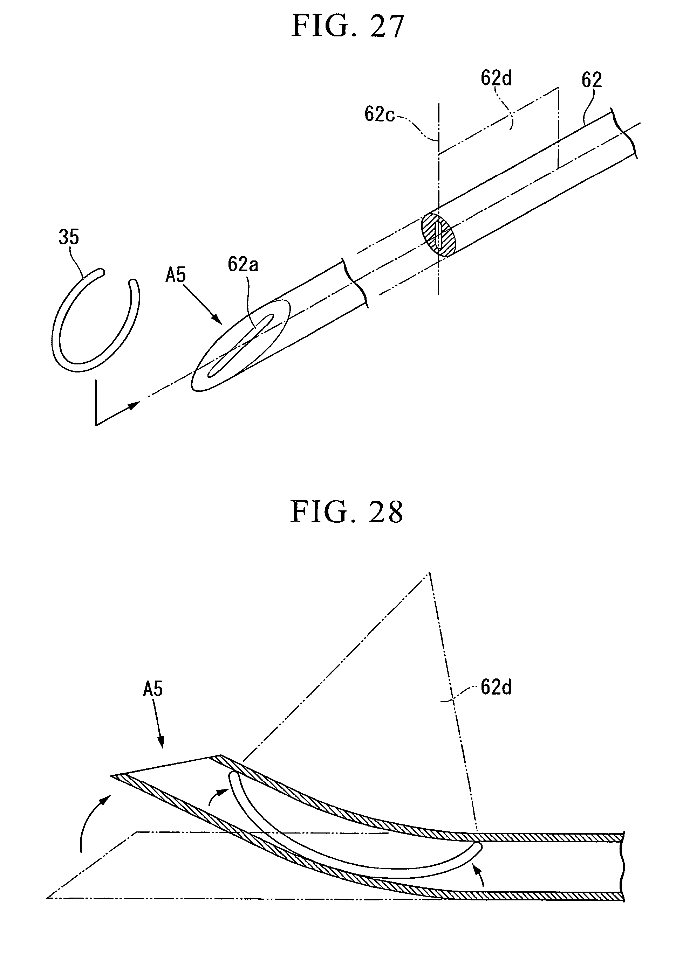

A needle tube 62 shown in FIG. 27 has an inner cavity formed in a flat cross-sectional shape, such as an ellipse, an oval, or a rectangle. The direction to visualize the largest area of an opening 62a in the needle tube (indicated by arrow A5 in FIG. 27) is substantially parallel with a longitudinal cross-sectional plane 62d that contains the long-diameter direction 62c of the flat shape.

The implant 35 is stored in the needle tube 62. Since the implant 35 attempts to return to its original shape inside the needle tube 62, it is stored such that it stretches in the long-diameter direction in the inner cavity.

On the other hand, the needle tube 62 has a certain amount of flexibility, and the force of the implant 35 attempting to return to its original shape is strong enough to make the needle tube 62 curve. Consequently, as shown in FIG. 28, the section of the needle tube 62 which the implant 35 is stored in (i.e. the region near the distal end of the needle tube 62) curves in a smooth circular-arc shape in the longitudinal cross-sectional plane 62d. As a result, while the size of the circular-arc shape region of the needle tube 62 depends on the size of the implant 35, in external appearance it has a shape broadly similar to that of the needle tube 34 shown in FIG. 12.

Thus in the present example, as in the first example, the implant 35 can be favorably confirmed on the ultrasonic image.

As described above, according to the examples and the embodiment of the present invention, by matching the angular direction position of the needle tube opening part to the ultrasonic scan plane of the ultrasonic endoscope, it is possible to provide the puncture needle for ultrasound biopsy capable of accurately delivering a substance to a region of interest while favorably observing the delivery of the substance with the ultrasonic endoscope.

In addition, the present invention is not limited to the embodiment described above, and can be modified in various ways without departing from the scope of the present invention.

### Industrial Applicability

According to the puncture needle for ultrasound biopsy used in combination with the ultrasonic endoscope, a substance can be accurately delivered to a region of interest while the delivery of the substance is favorably being observed with the ultrasonic endoscope by matching the angular direction position of the needle tube opening part to the ultrasonic scan plane of the ultrasonic endoscope.

### Brief Description of the Referenced Symbols

- 1, 1':: ultrasonic endoscope
- 2, 31:: insertion part
- 2a, 2a':: distal-end hard part
- 2b:: bendable part
- 2c:: flexible tube part
- 3, 32:: operation part
- 3a:: angle knob
- 3d:: treatment tool insertion hole
- 4:: universal cord
- 5:: endoscope connector
- 6:: ultrasonic cable
- 7:: ultrasonic connector
- 10, 10':: ultrasonic transducer part
- 10A, 10A':: ultrasonic scan plane
- 21:: distal-end face
- 22:: observation window
- 23:: illumination window
- 24, 24':: insertion channel exit
- 27:: insertion channel
- 30:: puncture needle for ultrasound biopsy
- 32:: operation part
- 33:: sheath
- 34, 54, 55, 60:: needle tube
- 34a, 54a, 55a, 60a:: opening
- 34b, 51, 34e:: plane
- 34d:: point
- 35:: implant
- 36:: stylet
- 36a:: knob
- 37:: operation-part main unit
- 37a:: flange part
- 37b:: notched step
- 38:: slider
- 39:: stopper
- 39a:: stopper member
- 39b:: fixing screw (stopper screw)
- 40:: connection part
- 40a:: screw
- 40b:: recess
- 40c:: distal-end connection part
- 41:: O-ring
- 42:: sliding arrangement member
- 43:: ferrule member
- 44:: guide pipe
- 50:: body-cavity tissue
- 52:: injection syringe
- 52a:: cylinder ferrule
- 52b:: cylinder
- 52c:: piston
- 53:: medicine
- 56, 57:: ultrasonic reflection machining
- 60c:: thinnest part
- 60d:: thickest part
- 60e:: line
- 60f:: cross-sectional plane
- A1, A2, A3, A4, A5:: direction
- L2, L2':: center axis
- L3, L3':: center line

## Claims

1. A puncture needle (30) for ultrasound biopsy used in combination with an ultrasonic endoscope (1,1') including a distal-end hard part (2a, 2a'), a bendable part (2b) which is connected to a proximal end of the distal-end hard part (2a, 2a') and which is bendable, and an operation part (3) which performs bending operation of the bendable part (2b), the puncture needle (30) comprising:
a sheath (33) that is inserted into an insertion channel (27) of the ultrasonic endoscope (1, 1') so as to be capable of advancing and retracting;
a needle tube (60) that has a predetermined outer diameter so as to be inserted into the sheath (33), and a sharp distal end configured to puncture a tissue (50) in a body cavity; and
a releasing mechanism (52) that is fitted to a proximal-end side of the needle tube (60), and releases a substance (35, 53) filled therein from an opening (60a) of the needle tube to an outside, **characterized in that**
the needle tube (60) has an inner cavity which extends along a longitudinal axis of the needle tube (60) and which centers on a position displaced from a center of the outer diameter, a thinnest part (60c) in which a thickness of a border of the opening (60a), which is formed in a distal-end region of the needle tube (60) so as to communicate with the inner cavity, is the thinnest, and a thickest part (60d) in which the thickness is the thickest,
a direction (A3) of the opening (60a) of the needle tube (60) is substantially parallel with a cross-sectional plane (60f) connecting the thinnest part (60c) and the thickest part (60d),
in a state in which the needle tube (60) is disposed inside the bendable part (2b), a periphery of the needle tube (60) receives a force from an inner wall of the insertion channel (27) while the bendable part (2b) is in a bent state by the operation part (3), and
the needle tube (60) is rotated around a longitudinal axis of the insertion channel (27) by the force, such that the thinnest part (60c) is disposed on an inside of the bendable part (2b) and an axis line matching the direction (A3) of the opening (60a) in the needle tube protruding from the insertion channel (27) becomes substantially parallel with an ultrasonic scanning face (10A, 10A') of the ultrasonic endoscope (1, 1').

2. The puncture needle (30) for ultrasound biopsy according to Claim 1, wherein a most proximal-end side of the opening (60a) of the needle tube is in a cross-sectional plane (60f) connecting the thinnest part (60c) and the thickest part (60d).

3. The puncture needle (30) for ultrasound biopsy according to Claim 1, wherein the distal-end region covers the distal end.

4. The puncture needle (30) for ultrasound biopsy according to Claim 1, wherein the distal-end region covers a region near the distal end.

5. The puncture needle (30) for ultrasound biopsy according to Claim 1, wherein the substance (53) includes gas.

6. The puncture needle (30) for ultrasound biopsy according to Claim 1, wherein the substance (53) includes liquid.

7. The puncture needle (30) for ultrasound biopsy according to Claim 1, wherein the substance (35) includes a solid.

8. The puncture needle (30) for ultrasound biopsy according to Claim 7 wherein the solid includes a slender elastic body.

## Patentansprüche

1. Punktionskanüle (30) zur Ultraschallbiopsie, die in Kombination mit einem Ultraschallendoskop (1, 1') benutzt wird, das ein hartes distales Endteil (2a, 2a'), ein biegbares Teil (2b), das mit einem proximalen Ende des harten distalen Endteils (2a, 2a') verbunden ist und das biegbar ist, und ein Betätigungsteil (3) umfasst, das eine Biegebetätigung des biegbaren Teils (2b) durchführt, wobei die Punktionskanüle (30) umfasst:
eine Hülse (33), die in einen Einführkanal (27) des Ultraschallendoskops (1, 1') so eingeführt ist, dass sie in der Lage ist, vorgeschoben und zurückgezogen zu werden;
einen Nadelkanal (60), der einen vorbestimmten äußeren Durchmesser, so dass er in die Hülse (33) eingeführt werden kann und ein scharfes distales Ende aufweist, das dazu eingerichtet ist, ein Gewebe (50) in einer Körperhöhlung zu punktieren; und
einen Auslösemechanismus (52), der an einer proximalen Endseite des Nadelkanals (60) angebracht ist und eine darin eingefüllte Substanz (35, 53) von einer Öffnung (60a) des Nadelkanals nach außen freisetzt, **dadurch gekennzeichnet, dass**
der Nadelkanal (60) einen inneren Hohlraum, der sich entlang einer longitudinalen Achse des Nadelkanals (60) erstreckt und der sich an einer Position ausrichtet, die von einem Zentrum des äußeren Durchmessers verschoben ist, einen dünnsten Teil (60c), in dem eine Dicke eines Rands der Öffnung (60a), die in einem distalen Endbereich des Nadelkanals (60) ausgebildet ist, um mit dem inneren Hohlraum zu kommunizieren, die dünnste ist, und einen dicksten Teil (60d) aufweist, in dem die Dicke am dicksten ist,
eine Richtung (A3) der Öffnung (60a) des Nadelkanals (60) im Wesentlichen parallel zu einer Querschnittsfläche (60f) ist, die den dünnsten Teil (60c) und den dicksten Teil (60d) verbindet,
in einem Zustand, in dem der Nadelkanal (60) innerhalb des biegbaren Teils (2b) angeordnet ist, ein Außenbereich des Nadelkanals (60) eine Kraft von einer Innenwand des Einführkanals (27) aufnimmt, während das biegbare Teil (2b) durch das Betätigungsteil (3) gebogen ist, und
der Nadelkanal (60) durch die Kraft um eine longitudinale Achse des Einführkanals (27) so rotiert wird, dass der dünnste Teil (60c) an einer Innenseite des biegbaren Teils (2b) angeordnet ist und eine Achslinie, die mit der Richtung (A3) der Öffnung (60a) in dem Nadelkanal, der von dem Einführkanal (27) hervorsteht, übereinstimmt, im Wesentlichen parallel mit einer Ultraschallscanfläche (10A, 10A') des Ultraschallendoskops (1, 1') wird.

2. Punktionskanüle (30) zur Ultraschallbiopsie gemäß Anspruch 1, wobei eine am meisten proximale Endseite der Öffnung (60a) des Nadelkanals in einer Querschnittsfläche (60f) liegt, die den dünnsten Teil (60c) und den dicksten Teil (60d) verbindet.

3. Punktionskanüle (30) zur Ultraschallbiopsie gemäß Anspruch 1, wobei der distale Endbereich das distale Ende überdeckt.

4. Punktionskanüle (30) zur Ultraschallbiopsie gemäß Anspruch 1, wobei der distale Endbereich einen Bereich in der Nähe des distalen Endes überdeckt.

5. Punktionskanüle (30) zur Ultraschallbiopsie gemäß Anspruch 1, wobei die Substanz (53) ein Gas umfasst.

6. Punktionskanüle (30) zur Ultraschallbiopsie gemäß Anspruch 1, wobei die Substanz (53) eine Flüssigkeit umfasst.

7. Punktionskanüle (30) zur Ultraschallbiopsie gemäß Anspruch 1, wobei die Substanz (35) einen Festkörper umfasst.

8. Punktionskanüle (30) zur Ultraschallbiopsie gemäß Anspruch 7, wobei der Festkörper einen schmalen elastischen Körper umfasst.

## Revendications

1. Aiguille de ponction (30) pour biopsie par ultrasons utilisée en combinaison avec un endoscope à ultrasons (1, 1') contenant une partie rigide d'extrémité distale (2a, 2a'), une partie cintrable (2b) qui est connectée à une extrémité proximale de la partie rigide d'extrémité distale (2a, 2a') et qui est cintrable, et une partie d'actionnement (3) qui réalise une opération de cintrage de la partie cintrable (2b), l'aiguille de ponction (30) comprenant :
une gaine (33) qui est insérée dans un canal d'insertion (27) de l'endoscope à ultrasons (1, 1') de façon à être capable d'avancer et de reculer ;
un tube d'aiguille (60) qui présente un diamètre externe prédéterminé de façon à être inséré dans la gaine (33), et une extrémité distale pointue configurée pour percer un tissu (50) dans une cavité de corps ; et
un mécanisme de libération (52) qui est ajusté sur un côté d'extrémité proximale du tube d'aiguille (60), et libère une substance (35, 53) remplie dans celui-ci à partir d'une ouverture (60a) du tube d'aiguille vers un extérieur, **caractérisé en ce que**
le tube d'aiguille (60) comporte une cavité interne qui s'étend le long d'un axe longitudinal du tube d'aiguille (60) et qui est centrée sur une position déplacée par rapport à un centre du diamètre externe, une partie la plus fine (60c) dans laquelle une épaisseur d'une bordure de l'ouverture (60a), qui est formée dans une région d'extrémité distale du tube d'aiguille (60) de façon à communiquer avec la cavité interne, est la plus mince, et une partie la plus épaisse (60d) dans laquelle l'épaisseur est la plus épaisse,
une direction (A3) de l'ouverture (60a) du tube d'aiguille (60) est sensiblement parallèle à un plan en section transversale (60f) connectant la partie la plus fine (60c) et la partie la plus épaisse (60d),
dans un état dans lequel le tube d'aiguille (60) est disposé à l'intérieur de la partie cintrable (2b), une périphérie du tube d'aiguille (60) reçoit une force provenant d'une paroi interne du canal d'insertion (27) alors que la partie cintrable (2b) se trouve dans un état cintré par la partie d'actionnement (3), et
le tube d'aiguille (60) est tourné autour d'un axe longitudinal du canal d'insertion (27) par la force, d'une manière telle que la partie la plus fine (60c) est disposée sur un intérieur de la partie cintrable (2b) et une ligne axiale correspondant à la direction (A3) de l'ouverture (60a) dans le tube d'aiguille faisant saillie depuis le canal d'insertion (27) devient sensiblement parallèle à une face de balayage ultrasonore (10A, 10A') de l'endoscope à ultrasons (1, 1').

2. Aiguille de ponction (30) pour biopsie par ultrasons selon la revendication 1, dans laquelle le côté d'extrémité le plus proximal de l'ouverture (60a) du tube d'aiguille se trouve dans un plan en section transversale (60f) connectant la partie la plus fine (60c) et la partie la plus épaisse (60d).

3. Aiguille de ponction (30) pour biopsie par ultrasons selon la revendication 1, dans laquelle la région d'extrémité distale couvre l'extrémité distale.

4. Aiguille de ponction (30) pour biopsie par ultrasons selon la revendication 1, dans laquelle la région d'extrémité distale couvre une région proche de l'extrémité distale.

5. Aiguille de ponction (30) pour biopsie par ultrasons selon la revendication 1, dans laquelle la substance (53) inclut du gaz.

6. Aiguille de ponction (30) pour biopsie par ultrasons selon la revendication 1, dans laquelle la substance (53) inclut du liquide.

7. Aiguille de ponction (30) pour biopsie par ultrasons selon la revendication 1, dans laquelle la substance (53) inclut un solide.

8. Aiguille de ponction (30) pour biopsie par ultrasons selon la revendication 7, dans laquelle le solide inclut un corps élastique élancé.
